# EUROPEAN PATENT APPLICATION

(11) **EP 4 230 064 A1**
(43) Date of publication of application: **23.08.2023**
(21) Application number: 22214815.7
(22) Date of filing: 20.12.2022
(51) Int. Cl.: A24F 40/05, A24F 40/10, A24F 40/40, A24F 40/50, A61M 15/00

(54) **MICROPOROUS ATOMIZATION ASSEMBLY AND ULTRASONIC ATOMIZATION DEVICE**

(30) Priority: 21.12.2021 CN 202111571341
(71) Applicant: Shenzhen Moore Vaporization Health & Medical Technology Co., Ltd., Shenzhen, Guangdong (CN)
(72) Inventor: LIU, Changqing, Shenzhen, Guangdong (CN)
(74) Representative: De Arpe Tejero, Manuel

(57) **Abstract**

This present disclosure discloses a microporous atomization assembly (1) and an ultrasonic atomization device. The microporous atomization assembly (1) includes a microporous atomization plate (10) and a circuit board (20). The microporous atomization plate (10) is configured to atomize an aerosol-generation substrate and generate aerosols; and the circuit board (20) is flexibly electrically connected to the microporous atomization plate (10), and is configured to transmit an electrical signal of the circuit board (20) to the microporous atomization plate (10). By connecting the circuit board (20) and the microporous atomization plate (10) through a flexible material, the problems of attenuation of performance parameters of the atomization plate, poor product consistency, and a high scrap rate caused by a soldering iron heated and soldered on the microporous atomization plate (10) in the related art are resolved.

## Description

### CROSS-REFERENCE TO PRIOR APPLICATION

Priority is claimed to Chinese Patent Application No. CN 202111571341.0, filed on December 21, 2021, the entire disclosure of which is hereby incorporated by reference herein.

### TECHNICAL FIELD

This present disclosure relates to the field of atomization technologies, and specifically, to a microporous atomization assembly and an ultrasonic atomization device.

### BACKGROUND

In the related art, an ultrasonic atomization device includes a power supply assembly and a microporous atomization assembly. An electrical signal wire of an atomization plate of the microporous atomization assembly is heated and soldered on an atomization ceramic plate by a soldering iron, leading to attenuation of performance parameters of the atomization plate and poor product consistency.

### SUMMARY

In view of this, this present disclosure provides a microporous atomization assembly and an ultrasonic atomization device, to resolve the problems of attenuation of performance parameters of an atomization plate and poor product consistency caused by a soldering iron heated and soldered on an atomization ceramic plate in the related art.

To resolve the foregoing technical problems, a first technical solution adopted by this present disclosure is to provide a microporous atomization assembly, including a microporous atomization plate and a circuit board. The microporous atomization plate is configured to atomize an aerosol-generation substrate and generate aerosols; and the circuit board is flexibly electrically connected to the microporous atomization plate, and is configured to transmit an electrical signal of the circuit board to the microporous atomization plate.

In an embodiment, the microporous atomization assembly further includes a flexible electrical conductor arranged between the circuit board and the microporous atomization plate, and the flexible electrical conductor is in contact with the circuit board and the microporous atomization plate respectively.

In an embodiment, the circuit board and the microporous atomization plate are fixedly connected through viscosity of the flexible electrical conductor or an adhesion layer on a surface of the flexible electrical.

In an embodiment, the microporous atomization assembly further includes a clamping component. The circuit board and the microporous atomization plate are fixedly connected through the clamping component.

In an embodiment, the clamping component includes a first rigid fixing member and a second rigid fixing member. The first rigid fixing member is arranged on one side of the microporous atomization plate. The second rigid fixing member is arranged on another side of the microporous atomization plate and clamping the circuit board and the microporous atomization plate by cooperating with the first rigid fixing member.

In an embodiment, the circuit board is arranged between the microporous atomization plate and the first rigid fixing member; the second rigid fixing member is provided with a first through-hole; and the microporous atomization assembly further includes an electrical conductive member arranged in the first through-hole. One end of the electrical conductive member is electrically connected to the circuit board, and another end of the electrical conductive member is configured to connect to an external wire.

In an embodiment, a surface of the second rigid fixing member away from the first rigid fixing member is provided with a first groove, and the first through-hole is provided on a bottom wall of the first groove; the electrical conductive member includes a connection portion and an extending portion, and a diameter of the connection portion is greater than a diameter of the extending portion; and the connection portion is connected to the first groove, and the extending portion is clamped in the first through-hole.

In an embodiment, the clamping component further includes: a first flexible fixing member and a second flexible fixing member. The first flexible fixing member is arranged between the first rigid fixing member and the microporous atomization plate. The second flexible fixing member is arranged between the second rigid fixing member and the microporous atomization plate.

In an embodiment, the microporous atomization plate includes a metal baseplate. The plurality of atomization holes are provided on the metal baseplate; and the first rigid fixing member, the second rigid fixing member, the first flexible fixing member, and the second flexible fixing member are all annular bodies, and the plurality of atomization holes are exposed through through-holes of the annular bodies.

In an embodiment, a bottom surface and a side surface of the microporous atomization plate are both fixed in the first flexible fixing member and the second flexible fixing member.

In an embodiment, the first rigid fixing member includes a first ring-shaped body. The first ring-shaped body includes a first surface and a second surface arranged opposite to each other. The first surface is a surface far away from the microporous atomization plate. The second surface is a surface close to the microporous atomization plate. The second surface of the first ring-shaped body includes a second groove configured to accommodate the first flexible fixing member.

In an embodiment, the first flexible fixing member is an annular body with a smooth surface, or is a coarse annular body whose surface including a plurality of protruding points or bumps.

In an embodiment, the microporous atomization assembly further includes a ring-shaped seal member arranged at a joint of the first rigid fixing member and the second rigid fixing member and attached to the joint of the first rigid fixing member and the second rigid fixing member.

In an embodiment, the flexible electrical conductor is an electrical conductive rubber or electrical conductive foam.

To resolve the foregoing technical problems, a second technical solution adopted by this present disclosure is to provide an ultrasonic atomization device, including a microporous atomization assembly, a power supply assembly, and a shell. The microporous atomization assembly is the microporous atomization assembly according to any one of the foregoing; the power supply assembly is connected to the microporous atomization assembly and configured to supply power to the microporous atomization assembly; and the microporous atomization assembly is arranged on the shell, the shell cooperates with the microporous atomization assembly to form a liquid storage tank, and the liquid storage tank is configured to store an aerosol-generation substrate.

Beneficial effects of this present disclosure are as follows: Different from the related art, the microporous atomization assembly in this present disclosure includes a microporous atomization plate and a circuit board. The microporous atomization plate is configured to atomize an aerosol-generation substrate to generate aerosols; and the circuit board is flexibly electrically connected to the microporous atomization plate, to transmit an electrical signal of the circuit board to the microporous atomization plate. By connecting the circuit board and the microporous atomization plate through a flexible material, the problems of attenuation of performance parameters of the atomization plate and poor product consistency caused by a soldering iron heated and soldered on the microporous atomization plate in the related art are resolved. In addition, the flexible electrical connection may greatly buffer force generated by vibration atomization of the microporous atomization plate, so that force acting on the microporous atomization plate may be omitted, and the microporous atomization plate may perform circumferential and radial vibration at a fixedly frequency without attenuation, thereby having a good atomization effect.

### BRIEF DESCRIPTION OF THE DRAWINGS

To describe the technical solutions in embodiments of this present disclosure more clearly, the following briefly introduces the accompanying drawings required for describing the embodiments. Apparently, the accompanying drawings in the following descriptions show merely some embodiments of this present disclosure, and a person of ordinary skill in the art may still derive other accompanying drawings from these accompanying drawings without creative efforts.
FIG. 1 is a schematic structural view of an ultrasonic atomization device according to this present disclosure.
FIG. 2 is a schematic exploded structural view of a microporous atomization assembly according to an embodiment of this present disclosure.
FIG. 3 is a front cross-sectional view of a microporous atomization assembly according to an embodiment of this present disclosure.
FIG. 4 is a schematic view of a specific structure of the microporous atomization assembly in FIG. 2.
FIG. 5 is a schematic structural view of a first rigid fixing member in FIG. 2 from a first perspective.
FIG. 6 is a schematic structural view of a first rigid fixing member in FIG. 2 from a second perspective.
FIG. 7 is a schematic structural view of a second rigid fixing member in FIG. 2 from a first perspective.
FIG. 8 is a schematic structural view of a second rigid fixing member in FIG. 2 from a second perspective.
FIG. 9 is a schematic structural view of a second flexible fixing member in FIG. 2 from a first perspective.
FIG. 10 is a schematic structural view of a second flexible fixing member in FIG. 2 from a second perspective.
FIG. 11 is a schematic structural view of a circuit board according to an embodiment of this present disclosure.

### DETAILED DESCRIPTION

The technical solutions in embodiments of this present disclosure are clearly and completely described below with reference to the accompanying drawings in the embodiments of this present disclosure. Apparently, the described embodiments are merely some rather than all of the embodiments of this present disclosure. All other embodiments obtained by a person of ordinary skill in the art based on the embodiments of this present disclosure without creative efforts shall fall within the protection scope of this present disclosure.

The terms "first" and "second" in this present disclosure are merely intended for a purpose of description, and shall not be understood as indicating or implying relative significance or implicitly indicating the number of indicated technical features. Therefore, features defining "first" or "second" may explicitly or implicitly include at least one of the features. All directional indications (for example, upper, lower, left, right, front, and rear) in the embodiments of this present disclosure are merely used for explaining relative position relationships, movement situations, or the like between the various components in a specific posture (as shown in the accompanying drawings). If the specific posture changes, the directional indications change accordingly. In addition, the terms "include", "have", and any variant thereof are intended to cover a non-exclusive inclusion. For example, a process, method, system, product, or device that includes a series of steps or units is not limited to the listed steps or units, but further optionally includes a step or unit that is not listed, or further optionally includes another step or unit that is intrinsic to the process, method, product, or device.

"Embodiment" mentioned in this specification means that particular features, structures, or characteristics described with reference to the embodiment may be included in at least one embodiment of this present disclosure. The term appearing at different positions of this specification may not refer to the same embodiment or an independent or alternative embodiment that is mutually exclusive with another embodiment. A person skilled in the art explicitly or implicitly understands that the embodiments described in this specification may be combined with other embodiments.

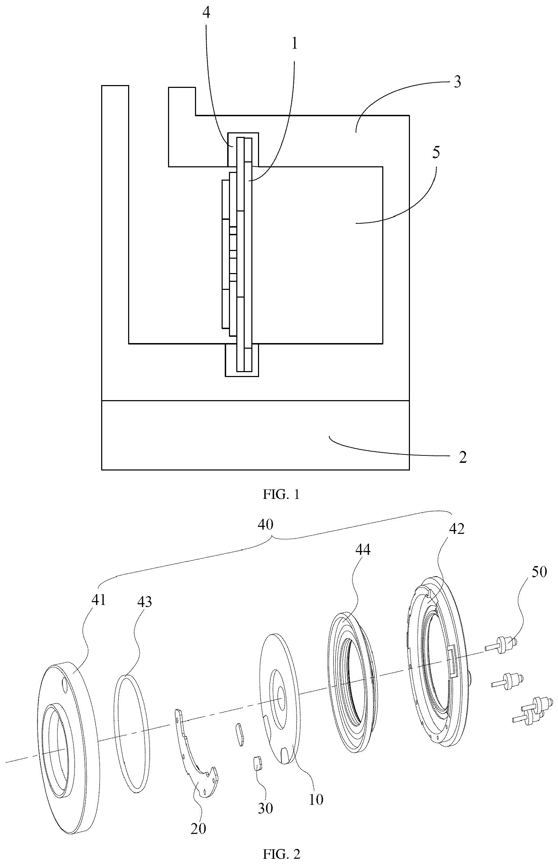

Referring to FIG. 1, FIG. 1 is a schematic structural view of an ultrasonic atomization device according to this present disclosure.

The ultrasonic atomization device may be configured to atomize a liquid substrate and includes a microporous atomization assembly 1, a power supply assembly 2, and a shell 3. The power supply assembly 2 is electrically connected to the microporous atomization assembly 1 and configured to supply power to the microporous atomization assembly 1. The microporous atomization assembly 1 is arranged on the shell 3, a closed region formed by cooperation between the shell 3 and the microporous atomization assembly 1 is a liquid storage tank 5, and the liquid storage tank 5 is configured to store an aerosol-generation substrate. A shape and a size of the liquid storage tank 5 are not limited, which may be designed as required. The power supply assembly 2 is fixedly connected to the shell 3. Under driving of the power supply assembly 2, the microporous atomization assembly 1 vibrates to atomize the liquid aerosol-generation substrate in the liquid storage tank 5 to form aerosols that can be inhaled by a user. The liquid aerosol-generation substrate may be liquid substrate such as medical liquid, an aerosol-generation substrate such as plant leaves or the like. The microporous atomization assembly 1 specifically may be applied in different fields, such as medical card, cosmetology, recreation inhalation or the like. The power supply assembly 2 includes a battery (not shown in the figure), an airflow sensor (not shown in the figure), and a controller (not shown in the figure). The battery is configured to supply power to the microporous atomization assembly 1, so that the microporous atomization assembly 1 can atomize the aerosol-generation substrate to form aerosols. The airflow sensor is configured to detect airflow changes in the ultrasonic atomization device, and the controller is configured to start the ultrasonic atomization device according to the airflow changes detected by the airflow sensor. The shell 3 and the power supply assembly 2 may be integrally arranged or may be detachably connected, which are designed according to a specific requirement. In this embodiment, the shell 3 and the power supply assembly 2 are detachably connected, and the microporous atomization assembly 1 and the power supply assembly 2 are also detachably connected. For example, the shell 3 and the power supply assembly 2 may be connected through a buckle, or a magnetic connector is arranged in the shell 3 and the power supply assembly 2 respectively to implement magnetic attraction connection, and the microporous atomization assembly 1 and the power supply assembly 2 may be connected through an electrode pin.

A shape and a material of the shell 3 are not limited, and the shell may be made of metal such as aluminum, stainless steel or the like, provided that the shell is not deteriorated due to reaction with the aerosol-generation substrate. In an embodiment, a ring-shaped groove 4 is provided on an inner side wall of the shell 3, and an edge of the microporous atomization assembly 1 is embedded in the ring-shaped groove 4 and is fixed to the ring-shaped groove 4 through adhesive.

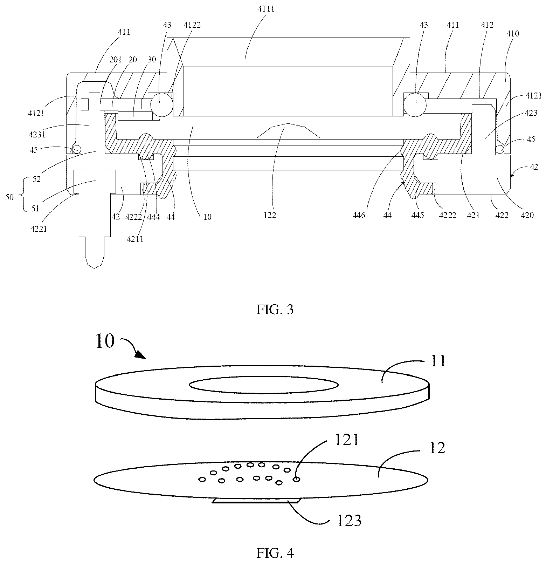

Referring to FIG. 2 and FIG. 3, FIG. 2 is a schematic exploded structural view of a microporous atomization assembly according to an embodiment of this present disclosure, and FIG. 3 is a front cross-sectional view of a microporous atomization assembly according to an embodiment of this present disclosure.

The microporous atomization assembly 1 includes a microporous atomization plate 10, a circuit board 20, a flexible electrical conductor 30, a clamping component 40, and an electrical conductive member 50. The microporous atomization plate 10 is configured to atomize the aerosol-generation substrate. The circuit board 20 is flexibly electrically connected to the microporous atomization plate 10, and is configured to transmit an electrical signal of the circuit board 20 to the microporous atomization plate 10. The flexible electrical conductor 30 is arranged between the circuit board 20 and the microporous atomization plate 10, and the flexible electrical conductor 30 is in contact with the circuit board 20 and the microporous atomization plate 10 respectively, to implement a flexible electrical connection between the circuit board 20 and the microporous atomization plate 10. The circuit board 20 and the microporous atomization plate 10 may be fixedly connected through the clamping component 40 or fixedly connected in another manner. The electrical conductive member 50 is electrically connected to the circuit board 20 and is connected to the power supply assembly 2.

Referring to FIG. 4, FIG. 4 is a schematic view of a specific structure of the microporous atomization assembly in FIG. 2.

In an embodiment, the microporous atomization plate 10 is a ceramic atomization plate. The microporous atomization plate 10 includes a piezoelectric ceramic plate 11 and a metal baseplate 12 that are stacked. A first electrode layer is arranged on a side surface of the piezoelectric ceramic plate 11 away from the metal baseplate 12, and the electrode layer is generally made of a material with high electrical conductive performance, for example, a metal material including silver or copper. A second electrode layer may also be arranged on a side surface of the piezoelectric ceramic plate 11 close to the metal baseplate 12. The second electrode layer extends to a plane in which the first electrode layer is arranged. An advantage of this method lies in that an electrical connection between positive and negative electrodes may be implemented by arranging two flexible electrical conductors 30 on the same side. In some other implementations, the metal baseplate 12 may be used as another electrode in cooperation with the first electrode layer. The circuit board 20 and the flexible electrical conductor 30 are arranged on a surface of the piezoelectric ceramic plate 11 away from the metal baseplate 12.

Specifically, as shown in FIG. 3 and FIG. 4, the piezoelectric ceramic plate 11 is in a shape of a ring, such as a shape of a circular ring, the metal baseplate 12 covers a through-hole of the ring-shaped piezoelectric ceramic plate 11, a protruding portion 122 is arranged at a center of the metal baseplate 12, the protruding portion 122 protrudes toward the piezoelectric ceramic plate 11 and is exposed through the through-hole of the piezoelectric ceramic plate 11, the protruding portion 122 is provided with a plurality of micropores, and the plurality of micropores are used as atomization holes 121 of the microporous atomization plate 10. The microporous atomization plate 10 vibrates and atomizes the aerosol-generation substrate from the atomization holes 121 and generates aerosols, and the aerosols run through the atomization holes 121 to be inhaled by the user. Further, a positioning portion 123 is arranged on a surface of the metal baseplate 12 away from the protruding portion 122, and the positioning portion is configured to ensure that positions of the metal baseplate 12 and the piezoelectric ceramic plate 11 are positioned accurately. Shapes, sizes, and numbers of the piezoelectric ceramic plates 11 and the metal baseplates 12 may be set as required. A shape of the atomization hole 121 may be one or any combination of a cone, a rectangle, and a circle, provided that the aerosol-generation substrate can be atomized and exported, which is not limited in this present disclosure.

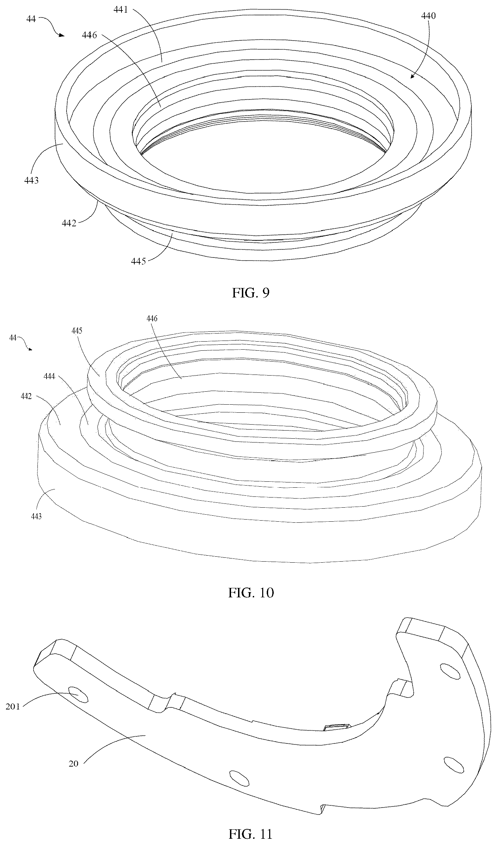

As shown in FIG. 2 and FIG. 11, FIG. 11 is a schematic structural view of a circuit board according to an embodiment of this present disclosure.

The circuit board 20 may be a printed circuit board (PCB) or a flexible printed circuit (FPC). In this embodiment, the circuit board 20 is a PCB, and the PCB is connected to the microporous atomization plate 10 through the flexible electrical conductor 30, to implement a flexible electrical connection with the microporous atomization plate 10. In this embodiment, the circuit board 20 is an arc-shaped PCB, which is entirely arranged on a surface of the piezoelectric ceramic plate 11. The circuit board 20 includes a positive contact and a negative contact, one flexible electrical conductor 30 is arranged between the positive contact and the piezoelectric ceramic plate 11, and another flexible electrical conductor 30 is arranged between the negative contact and the piezoelectric ceramic plate 11, and materials, shapes, and volumes of the two flexible electrical conductors 30 are totally the same. An outer diameter of the circuit board 20 is approximately flush with an outer diameter of the microporous atomization plate 10. That is, an outer diameter of the PCB may be exactly flush with the outer diameter of the microporous atomization plate 10, or may be longer than or shorter than the outer diameter of the microporous atomization plate 10 to some extent, provided that the outer diameter of the PCB does not exceed an outer side wall of the clamping component 40. In some other embodiments, the circuit board 20 may also be a flexible circuit board and partially bends to a side surface of the microporous atomization plate 10. Meanwhile, the PCB may also be in another shape, provided that the PCB matches and is connected to the microporous atomization plate 10, which is not limited in this present disclosure.

The circuit board 20 is in a flexible electrical connection to the microporous atomization plate 10. A difference between the flexible electrical connection and a connection manner such as a rigid connection or soldering lies in that, the circuit board 20 is connected to the microporous atomization plate 10 through a flexible material without soldering, so that a joint between the circuit board 20 and the microporous atomization plate 10 may be elastically deformed within a specific range. In another existing technology, an electrical signal of the microporous atomization plate 10 needs to transmitted by a metal elastic pin to the microporous atomization plate 10 to perform elastic contact, which applies great acting force on the microporous atomization plate 10. As a result, an electrical parameter of the microporous atomization plate 10 deviates from a theoretical value, for example, an impedance value is increased, a wave form is disordered, or a resistance value is increased. In addition, during operation of the microporous atomization plate 10, the metal elastic pin is in a static state, the microporous atomization plate 10 is in a high-frequency vibration state, a top protective layer of the metal elastic pin is easily worn out and oxidized, and a service life of the entire machine is greatly reduced. By using the flexible electrical connection manner in this present disclosure, force generated by vibration and atomization of the microporous atomization plate 10 may be greatly buffered, so that force acting on the microporous atomization plate may be omitted, thereby resolving the foregoing technical difficulty well. Meanwhile, a process of wire bonding through a soldering iron is omitted, so that assembly is simpler and costs are lower. In the related art, the electrical signal of the microporous atomization plate 10 is transmitted to the ceramic plate through a soldered wire. When a wire is soldered, a temperature of the soldering iron, a duration of a heating time, and a diameter size and a height of a pad are directly related to electrical performance parameters of the microporous atomization plate 10, for example, an impedance value is increased, a wave form is disordered, or a resistance value is increased, and these electrical performance parameters are directly related to an atomization amount of a product and a size of an atomized particle. Meanwhile, factors such as the temperature of the soldering iron, the duration of the soldering time, and the size and the height of the pad are uncontrollable during manual operations, which has a high requirement on experience of operators and can hardly ensure the consistency during batch production. The soldered wire of the microporous atomization plate 10 is not required in the technical solution of this present disclosure, so that the difficulty in the related art is resolved.

In an embodiment, as shown in FIG. 2, the flexible electrical conductor 30 is an electrical conductive plate made of a flexible electrical conductive material, and the flexible electrical conductive material may be a flexible electrical conductive rubber, electrical conductive foam or the like, or may be another flexible adhesive. The flexible electrical conductor 30 may be fixedly connected to the circuit board 20 and the microporous atomization plate 10 through viscosity of the flexible electrical conductor, or may be fixedly connected to the circuit board 20 and the microporous atomization plate 10 through an adhesion layer. The circuit board 20 and the microporous atomization plate 10 are fixedly connected through the viscosity of the flexible electrical conductor 30 or the adhesion layer on a surface of the flexible electrical conductor 30. Because the flexible electrical conductor 30 is made of a flexible material, the flexible electrical conductor may connect the circuit board 20 to the microporous atomization plate 10 more firmly, and elastic deformation to some extent may be generated among the microporous atomization plate 10, the circuit board 20, and the flexible electrical conductor 30. The adhesion layer may be an electrical conductive adhesive. In some other implementations, a limiting groove or a limiting rib may also be provided on the circuit board 20 or the clamping component 4 to fix the flexible electrical conductor 30. The flexible electrical conductor 30 may also be fixed between the circuit board 20 and the microporous atomization plate 10 through friction generated by elastic deformation of the flexible electrical conductor.

In an embodiment, as shown in FIG. 2 and FIG. 3, the clamping component 40 is configured to clamp the circuit board 20 and the microporous atomization plate 10, to implement a fixed connection between the circuit board 20 and the microporous atomization plate 10. The clamping component 40 includes a first rigid fixing member 41, a second rigid fixing member 42, a first flexible fixing member 43, and a second flexible fixing member 44. The first rigid fixing member 41, the second rigid fixing member 42, the first flexible fixing member 43, and the second flexible fixing member 44 are all annular bodies, and the plurality of atomization holes 121 are exposed through through-holes of the annular bodies, so that aerosols can run through the through-holes of the annular bodies. The first rigid fixing member 41 is arranged on one side of the microporous atomization plate 10, the second rigid fixing member 42 is arranged on another side of the microporous atomization plate 10, and the second rigid fixing member 42 clamps the circuit board 20 and the microporous atomization plate 10 by cooperating with the first rigid fixing member 41. The first flexible fixing member 43 is arranged between the first rigid fixing member 41 and the microporous atomization plate 10, the second flexible fixing member 44 is arranged between the second rigid fixing member 42 and the microporous atomization plate 10, and the first flexible fixing member 43 and the second flexible fixing member 44 are both configured to buffer force generated by vibration between the first rigid fixing member 41 or the second rigid fixing member 42 and the circuit board 20 and the microporous atomization plate 10. A bottom surface and a side surface of the microporous atomization plate 10 are both fixed in the first flexible fixing member 43 and the second flexible fixing member 44, so that the microporous atomization plate performs circumferential and radial vibration at a fixed frequency without attenuation, and the atomization effect of the microporous atomization plate 10 may not be affected.

Specifically, the circuit board 20 is arranged between the microporous atomization plate 10 and the first rigid fixing member 41, and one side of the circuit board 20 is fixed to the microporous atomization plate 10 through the first rigid fixing member 41. The first rigid fixing member 41 and the second rigid fixing member 42 are both annular bodies and are both made of a rigid fixing material such as ceramic, steel, iron or the like. The first rigid fixing member 41 and the second rigid fixing member 42 are fixed in a detachable manner such as buckling, clamping, threaded connection or the like. Therefore, on one hand, disassembly is facilitated, and on the other hand, positions of the circuit board 20 and the microporous atomization plate 10 are relatively fixedly maintained within a range formed after the first rigid fixing member 41 is connected to the second rigid fixing member 42, thereby preventing a relatively great displacement from being generated between the circuit board 20 and the microporous atomization plate 10.

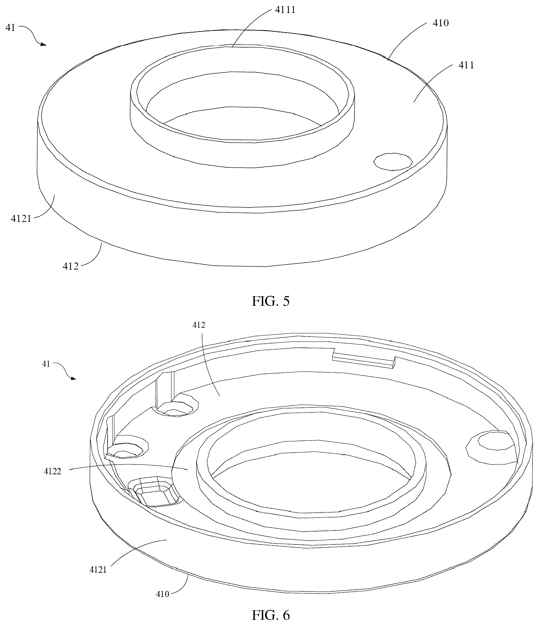

Referring to FIG. 3, FIG. 5, and FIG. 6, FIG. 5 is a schematic structural view of a first rigid fixing member in FIG. 2 from a first perspective; and FIG. 6 is a schematic structural view of a first rigid fixing member in FIG. 2 from a second perspective.

The first rigid fixing member 41 includes a first ring-shaped body 410, a first convex ring 4111, and a second convex ring 4121 arranged coaxially. The first ring-shaped body 410 includes a first surface 411 and a second surface 412 arranged opposite to each other, the first surface 411 is a surface away from the microporous atomization plate 10, and the second surface 412 is a surface close to the microporous atomization plate 10. The first convex ring 4111 protrudes toward a direction away from the first surface 411, the second convex ring 4121 protrudes toward a direction away from the second surface 412, and the first convex ring 4111 and the second convex ring 4121 protrude toward opposite directions and are both in a shape of a ring. An inner diameter of the first convex ring 4111 is the same as an inner diameter of the first ring-shaped body 410, thereby facilitating flowing of an aerosol substrate generated from the microporous atomization plate 10. An outer diameter of the second convex ring 4121 is the same as an outer diameter of the first ring-shaped body 410. In this embodiment, preferably, the second convex ring 4121 may extend along the outer diameter of the first ring-shaped body 410 in a direction away from the second surface 412 to a position connected to the second rigid fixing member 42. In this way, the first rigid fixing member 41 may be connected to the second rigid fixing member 42 more tightly, thereby improving the stability of the microporous atomization assembly 1 of this present disclosure. In some other embodiments, the second convex ring 4121 may extend along the outer diameter of the first ring-shaped body 410 in the direction away from the second surface 412 or may only extend to the outer diameter of the first ring-shaped body 410. An extending position and an extending direction of the second convex ring 4121 may also be set as required, which is not limited in this present disclosure.

Optionally, the second surface 412 of the first ring-shaped body 410 includes a second groove 4122 configured to accommodate the first flexible fixing member 43, so that vibration generated when the microporous atomization plate 10 atomizes the aerosol-generation substrate is greatly reduced when transmitted to the first rigid fixing member 41 through the first flexible fixing member 43. In addition, the vibration is buffered by the first flexible fixing member 43, so that the vibration generated by the microporous atomization plate 10 may not be immediately reduced when encountering the first rigid fixing member 41 but is gradually reduced through the first flexible fixing member 43. In this way, the vibration force generated by the microporous atomization plate 10 may be prevented from directly and frequently striking the first rigid fixing member 41 and causing a problem that the service life of the microporous atomization plate 10 is reduced since the microporous atomization plate 10 is worn out and oxidized, and the atomization effect of the microporous atomization plate 10 is also not affected.

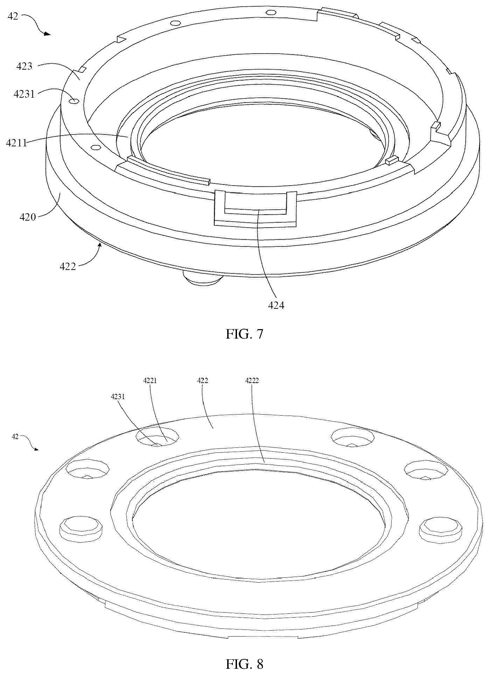

Referring to FIG. 2, FIG. 3, FIG. 7, and FIG. 8, FIG. 7 is a schematic structural view of a second rigid fixing member in FIG. 2 from a first perspective; and FIG. 8 is a schematic structural view of a second rigid fixing member in FIG. 2 from a second perspective.

Specifically, the second rigid fixing member 42 includes a second ring-shaped body 420 and a third convex ring 423 arranged coaxially. The second ring-shaped body 420 includes a third surface 421 and a fourth surface 422 arranged opposite to each other, the third surface 421 is a surface close to the first rigid fixing member 41, and the fourth surface 422 is a surface away from the first rigid fixing member 41. A side of the third surface 421 close to an outer diameter of the second ring-shaped body 420 is in contact with and connected to the second convex ring 4121 of the first rigid fixing member 41. The third surface 421 includes a third convex ring 423, and the third convex ring 423 protrudes toward a direction close to the first rigid fixing member 41. The second rigid fixing member 42 includes a first through-hole 4231, and the first through-hole 4231 runs through the second ring-shaped body 420 and the third convex ring 423. Preferably, the third surface 421 further includes a third groove 4211, and the third groove 4211 is configured to connect to the second flexible fixing member 44. Meanwhile, the second flexible fixing member 44 is correspondingly provided with a fourth convex ring 444, and the fourth convex ring 444 is connected to the third groove 4211 in a matching manner, to enhance the connection stability between the second flexible fixing member 44 and the second rigid fixing member 42. It may be understood that, the third groove 4211 may also be provided on the second flexible fixing member 44, and the fourth convex ring 444 may also be arranged on the third surface 421, provided that the matching connection between the second flexible fixing member 44 and the second rigid fixing member 42 can be implemented. In some other embodiments, the third groove 4211 and the fourth convex ring 444 in this present disclosure may not be arranged, which is not limited in this present disclosure.

Specifically, a side of the second convex ring 4121 of the first rigid fixing member 41 close to the second flexible fixing member 44 includes a plurality of buckles 424, and the plurality of buckles 424 are configured to connect to components such as the microporous atomization plate 10 and a temperature sensor (not shown in the figure). A specific size of the buckle 424 may be set as required, provided that the matching connection to the components such as the microporous atomization plate 10 and the temperature sensor can be implemented, which is not limited in this present disclosure.

Optionally, a ring-shaped seal member 45 is arranged at a joint of the first rigid fixing member 41 and the second rigid fixing member 42, and the seal member 45 is attached to the joint of the first rigid fixing member 41 and the second rigid fixing member 42 and seals a gap at the joint, so that the first rigid fixing member 41 is connected to the second rigid fixing member 42 more tightly, thereby improving the stability of the microporous atomization assembly 1 of this present disclosure. In this embodiment, the seal member 45 may be made of a flexible material such as foam, a rubber or the like. In this way, the connection sealing performance between the first rigid fixing member 41 and the second rigid fixing member 42 may be improved, and the first rigid fixing member 41 and the second rigid fixing member 42 may not be squeezed, thereby preventing an entire position of the clamping component 40 from moving.

Optionally, a first groove 4221 and a ring-shaped recess 4222 are defined on the fourth surface 422 of the second rigid fixing member 42, the first groove 4221 extends in a direction from the fourth surface 422 to the third surface 421, and a bottom wall of the first groove 4221 is in communication with the first through-hole 4231. A diameter of the first groove 4221 is greater than a diameter of the first through-hole 4231. The first groove 4221 and the first through-hole 4231 are configured to mount the electrical conductive member 50, and the ring-shaped recess 4222 is configured to connect to the second flexible fixing member 44.

As described above, the first flexible fixing member 43 is arranged between the microporous atomization plate 10 and the first rigid fixing member 41, and is clamped between the microporous atomization plate 10 and the first rigid fixing member 41 through the second groove 4122 of the first rigid fixing member 41. The first flexible fixing member 43 may be an annular body with a smooth surface, or may be a coarse annular body whose surface includes a plurality of protruding points or bumps. When the surface of the first flexible fixing member 43 is smooth, the surface may facilitate connection and mounting of the first flexible fixing member to the first rigid fixing member 41. When the surface of the first flexible fixing member 43 is coarse, friction between the surface and the microporous atomization plate 10 and the first rigid fixing member 41 may be increased, thereby improving the connection stability. The surface may be specifically selected as required, which is not limited in this present disclosure.

Referring to FIG. 2, FIG. 3, FIG. 9, and FIG. 10, FIG. 9 is a schematic structural view of a second flexible fixing member in FIG. 2 from a first perspective; and FIG. 10 is a schematic structural view of a second flexible fixing member in FIG. 2 from a second perspective.

In this embodiment, the second flexible fixing member 44 includes a third ring-shaped body 440, a fifth convex ring 443, and a sixth convex ring 445 arranged coaxially. The third ring-shaped body 440 includes a fifth surface 441 and a sixth surface 442 arranged opposite to each other, the fifth surface 441 is in contact with the microporous atomization plate 10, and the microporous atomization plate 10 at least partially covers the fifth surface 441, or the microporous atomization plate 10 totally covers the fifth surface 441. An outer diameter of the third ring-shaped body 440 is connected to the fifth convex ring 443 facing the first rigid fixing member 41, and the fifth convex ring 443 extends to a position close to the circuit board 20 and is arranged between the third convex ring 423 of the second rigid fixing member 42 and the microporous atomization plate 10. An inner diameter of the third ring-shaped body 440 is connected to the sixth convex ring 445 facing the fourth surface 422 of the second rigid fixing member 42. An outer diameter of the fifth convex ring 443 is the same as the outer diameter of the third ring-shaped body 440, and an inner diameter of the sixth convex ring 445 is the same as the inner diameter of the third ring-shaped body 440. Further, the sixth convex ring 445 may further transversely extend in a direction toward the fourth surface 422, and is clamped with the ring-shaped recess 4222 on the fourth surface 422. That is, the sixth convex ring 445 forms a corner extending toward the fourth surface 422, to wrap an inner side wall of the annular body of the second flexible fixing member 44 and the fourth surface 422, thereby implementing a protection function on the second rigid fixing member 42 to the greatest extent and preventing atomized liquid from leaking onto the second rigid fixing member 42. In some other embodiments, extending directions and extending positions of the fifth convex ring 443 and the sixth convex ring 445 may be set as required, which are not limited in this present disclosure.

Optionally, the inner wall of the annular body of the second flexible fixing member 44 may be provided with a plurality of annular protruding bodies 446, and the plurality of annular protruding bodies 446 may be configured to hermetically connect the microporous atomization assembly 1 to the liquid storage tank 5, thereby preventing liquid leakage in a process that the aerosol-generation substrate is guided into the atomization plate 10.

As shown in FIG. 3, in an embodiment, a material of the electrical conductive member 50 is metal such as copper or iron. An end of the electrical conductive member 50 runs through the first through-hole 4231 and is electrically connected to the circuit board 20, and the other end is configured to connect to an external wire.

Specifically, the electrical conductive member 50 includes a connection portion 51 and an extending portion 52, and a diameter of the connection portion 51 is greater than a diameter of the extending portion 52. The connection portion 51 is connected to the first groove 4221, and the extending portion 52 is clamped in the first through-hole 4231. In an embodiment, the connection portion 51 may be in a state that is basically flush with the fourth surface 422 of the second rigid fixing member 42 or may be in a state that apparently exceeds the fourth surface 422, provided that a connection to the external wire can be implemented, which is not limited in this present disclosure. Similarly, the extending portion 52 may extend to be exactly in contact with the circuit board 20 to be connected to the circuit board 20, or may extend to run through the circuit board 20 to be connected to the circuit board 20, which may be specifically set as required. When the extending portion 52 extends to run through the circuit board 20, the circuit board 20 is correspondingly provided with a second through-hole 201 matching the size and the number of the extending portion 52, to implement a matching connection between the circuit board 20 and the extending portion 52.

In this embodiment, as shown in FIG. 2, there are four electrical conductive members 50, two electrical conductive members are configured to connect to external wires, and the other two electrical conductive members are configured to connect to a temperature sensor (not shown in the figure). The temperature sensor is arranged on one end of the circuit board 20 close to the microporous atomization plate 10, and is arranged on the microporous atomization plate 10 through the circuit board 20. For example, the temperature sensor may be soldered on the circuit board 20. The temperature sensor is electrically connected to a controller (not shown in the figure) of the ultrasonic atomization device through the circuit board 20, the temperature sensor may measure a temperature of the microporous atomization plate 10, and the controller may control operations of the microporous atomization plate 10 according to a temperature measurement result fed back by the temperature sensor. The temperature sensor may be one or more of a positive temperature coefficient (PTC) thermal resistor, a negative temperature coefficient (NTC) thermal resistor, and a thermocouple, or may be another temperature sensing element. The temperature sensor accesses a control circuit to perform analog-to-digital converter (ADC) conversion processing, so that the temperature of the microporous atomization plate 10 may be obtained. The controller outputs a control signal according to the temperature of the microporous atomization plate 10 to change a voltage of an atomization driving voltage boost circuit, to implement automatic adjustment of atomization power.

In some other embodiments, the number and position of the electrical conductive member 50 may be set according to a specific requirement. For example, the electrical conductive member 50 may be arranged on the first surface 411 of the first rigid fixing member 41 and extend to the circuit board 20 to be connected to the circuit board 20, or may be arranged on an outer side wall of the first rigid fixing member 41 and connect the first rigid fixing member 41 and the circuit board 20, which are not limited in this present disclosure.

The microporous atomization assembly disclosed in this present disclosure includes a microporous atomization plate and a circuit board. The microporous atomization plate is configured to atomize an aerosol-generation substrate and generates aerosols; and the circuit board is flexibly electrically connected to the microporous atomization plate, and is configured to transmit an electrical signal of the circuit board to the microporous atomization plate. By connecting the circuit board and the microporous atomization plate through a flexible material, the problems of attenuation of performance parameters of the atomization plate, poor product consistency, and a high scrap rate caused by a soldering iron heated and soldered on the microporous atomization plate in the related art are resolved. In addition, the flexible electrical connection may greatly buffer force generated by vibration atomization of the microporous atomization plate, so that force acting on the microporous atomization plate may be omitted, and the microporous atomization plate may perform circumferential and radial vibration at a fixedly frequency without attenuation, thereby having a good atomization effect.

The foregoing descriptions are merely implementations of this present disclosure, and the patent scope of this present disclosure is not limited thereto. All equivalent structure or process changes made according to the content of this specification and accompanying drawings in this present disclosure or by directly or indirectly applying this present disclosure in other related technical fields shall fall within the protection scope of this present disclosure.

## Claims

1. A microporous atomization assembly (1), comprising:
a microporous atomization plate (10) configured to atomize an aerosol-generation substrate and generate aerosols; and
a circuit board (20) flexibly electrically connected to the microporous atomization plate (10), and is configured to transmit an electrical signal of the circuit board (20) to the microporous atomization plate (10).

2. The microporous atomization assembly (1) according to claim 1, further comprising:
a flexible electrical conductor (30) arranged between the circuit board (20) and the microporous atomization plate (10), wherein the flexible electrical conductor (30) is in contact with the circuit board (20) and the microporous atomization plate (10) respectively.

3. The microporous atomization assembly (1) as claimed in claim 2, wherein the circuit board (20) and the microporous atomization plate (10) are fixedly connected through viscosity of the flexible electrical conductor (30) or an adhesion layer on a surface of the flexible electrical conductor (30).

4. The microporous atomization assembly (1) as claimed in claim 2, further comprising:
a clamping component (40), wherein the circuit board (20) and the microporous atomization plate (10) are fixedly connected through the clamping component (40).

5. The microporous atomization assembly (1) as claimed in claim 4, wherein the clamping component (40) comprises:
a first rigid fixing member (41) arranged on one side of the microporous atomization plate (10); and
a second rigid fixing member (42) arranged on another side of the microporous atomization plate (10) and clamping the circuit board (20) and the microporous atomization plate (10) by cooperating with the first rigid fixing member (41).

6. The microporous atomization assembly (1) as claimed in claim 5, wherein the circuit board (20) is arranged between the microporous atomization plate (10) and the first rigid fixing member (41); the second rigid fixing member (42) is provided with a first through-hole (4231); and
the microporous atomization assembly (1) further comprises:
an electrical conductive member (50), arranged in the first through-hole (4231), wherein one end of the electrical conductive member (50) is electrically connected to the circuit board (20), and another end of the electrical conductive member (50) is configured to connect to an external wire.

7. The microporous atomization assembly (1) as claimed in claim 6, wherein a surface of the second rigid fixing member (42) away from the first rigid fixing member (41) is provided with a first groove (4221), and the first through-hole (4231) is provided on a bottom wall of the first groove (4221); the electrical conductive member (50) comprises a connection portion (51) and an extending portion (521), and a diameter of the connection portion (51) is greater than a diameter of the extending portion (52); and the connection portion (51) is connected to the first groove (4221), and the extending portion (52) is clamped in the first through-hole (4231).

8. The microporous atomization assembly (1) as claimed in claim 5, wherein the clamping component (40) further comprises:
a first flexible fixing member (43) arranged between the first rigid fixing member (41) and the microporous atomization plate (10); and
a second flexible fixing member (44) arranged between the second rigid fixing member (42) and the microporous atomization plate (10).

9. The microporous atomization assembly (1) as claimed in claim 8, wherein the microporous atomization plate (10) comprises:
a metal baseplate (12), wherein a plurality of atomization holes (121) are provided on the metal baseplate (12); and the first rigid fixing member (41), the second rigid fixing member (42), the first flexible fixing member (43), and the second flexible fixing member (44) are all annular bodies, and the plurality of atomization holes (121) are exposed through through-holes of the annular bodies.

10. The microporous atomization assembly (1) as claimed in claim 8, wherein a bottom surface and a side surface of the microporous atomization plate (10) are both fixed in the first flexible fixing member (43) and the second flexible fixing member (44).

11. The microporous atomization assembly (1) as claimed in claim 8, wherein the first rigid fixing member (41) comprises a first ring-shaped body (410);
wherein the first ring-shaped body (410) comprises a first surface (411) and a second surface (412) arranged opposite to each other, the first surface (411) is a surface far away from the microporous atomization plate (10), the second surface (412) is a surface close to the microporous atomization plate (10); and
wherein the second surface (412) of the first ring-shaped body (410) comprises a second groove (4122) configured to accommodate the first flexible fixing member (43).

12. The microporous atomization assembly (1) as claimed in claim 8, wherein the first flexible fixing member (43) is an annular body with a smooth surface, or is a coarse annular body whose surface comprising a plurality of protruding points or bumps.

13. The microporous atomization assembly (1) as claimed in claim 8, further comprising:
a ring-shaped seal member (45) arranged at a joint of the first rigid fixing member (41) and the second rigid fixing member (42) and attached to the joint of the first rigid fixing member (41) and the second rigid fixing member (42).

14. The microporous atomization assembly (1) as claimed in claim 2, wherein the flexible electrical conductor (30) is an electrical conductive rubber or electrical conductive foam.

15. An ultrasonic atomization device, comprising:
a microporous atomization assembly (1), wherein the microporous atomization assembly (1) is the microporous atomization assembly (1) as claimed in any one of claims 1 to 14;
a power supply assembly (2) connected to the microporous atomization assembly (1) and configured to supply power to the microporous atomization assembly (1); and
a shell (3), wherein the microporous atomization assembly (1) is arranged on the shell (3), the shell (3) cooperates with the microporous atomization assembly (1) to form a liquid storage tank (5), and the liquid storage tank (5) is configured to store an aerosol-generation substrate.
